# EUROPEAN PATENT SPECIFICATION

(11) **EP 2 164 980 B1**
(45) Date of publication and mention of the grant of the patent: **17.07.2013**
(21) Application number: 08758895.0
(22) Date of filing: 30.05.2008
(51) Int. Cl.: C12Q 1/26, G01N 33/573

(54) **BIOMARKER AND METHOD FOR DETERMINING AN OXIDATIVE STRESS LEVEL**
BIOMARKER UND VERFAHREN ZUR BESTIMMUNG EINES OXIDATIVEN STRESSNIVEAUS
BIOMARQUEUR ET PROCÉDÉ PERMETTANT DE DÉTERMINER UN NIVEAU DE STRESS OXYDATIF

(30) Priority: 31.05.2007 US 924821 P
(43) Date of publication of application: 24.03.2010
(60) Divisional application: 12185288.3
(73) Proprietor: BIOCRATES Life Sciences AG, 6020 Innsbruck (AT)
(72) Inventor: WEINBERGER, Klaus, Michael, A-64114 Mieming (AT); GRABER, Armin, A-6020 Innsbruck (AT); RAMSAY, Steven, Lewis, Heidelberg 3084 (AU)
(74) Representative: Hollatz, Christian
(86) International application number: PCT/EP2008/004324
(87) International publication number: WO 2008/145385

(56) References cited:
- WO-A-2007/003344
- ERLEMANN KARL-RUDOLF ET AL: "Regulation of 5-hydroxyeicosanoid dehydrogenase activity in monocytic cells" BIOCHEM. J.; BIOCHEMICAL JOURNAL APR 1 2007, vol. 403, no. 1, 1 April 2007 (2007-04-01), pages 157-165, XP002492795
- NECKAMEYER WENDI S ET AL: "Stress affects dopaminergic signaling pathways in Drosophila melanogaster." STRESS (AMSTERDAM, NETHERLANDS) JUN 2005, vol. 8, no. 2, June 2005 (2005-06), pages 117-131, XP008095634 ISSN: 1025-3890
- PINTO LUCAS S N M ET AL: "Dietary restriction protects against chronic-ethanol-induced changes in exploratory behavior in Wistar rats" BRAIN RESEARCH, vol. 1078, March 2006 (2006-03), pages 171-181, XP005364124 ISSN: 0006-8993
- MATALON R ET AL: "THE USE OF DEUTERATED PHENYL ALANINE FOR THE IN-VIVO ASSAY OF PHENYL ALANINE HYDROXYLASE ACTIVITY IN CHILDREN" JOURNAL OF INHERITED METABOLIC DISEASE, vol. 5, no. 1, 1982, pages 17-20, XP008095645 ISSN: 0141-8955
- SHORTLAND G J ET AL: "Phenylalanine kinetics in sick preterm neonates with Respiratory Distress syndrome" PEDIATRIC RESEARCH, vol. 36, no. 6, 1994, pages 713-718, XP008095644 ISSN: 0031-3998
- A BANAN ET AL: 'Ethanol-Induced Barrier Dysfunction and Its Prevention by Growth Factors in Human Intestinal Monolayers: Evidence for Oxidative and Cytoskeletal Mechanisms' THE JOURNAL OF PHARMACOLOGY AND EXPERIMENTAL THERAPEUTICS, [Online] 01 December 1999, pages 1075 - 1085, XP055016410 Retrieved from the Internet: <URL:http://jpet.aspetjournals.org/content/ 291/3/1075.full.pdf> [retrieved on 2012-01-12]

## Description

### Technical Field

This invention relates to a method for determining an oxidative stress level in a biological sample, which employs co-factor-dependent oxidative stress parameters. The co-factor is tetrahydrobiopterin.

### Background of the Invention

Oxidative stress measurement devices and methods have been described, for example, in WO 2006/90228, WO 2002/04029, WO 1999/63341, and EP 0 845 732.

Free radicals are atoms or molecules containing unpaired electrons. It is commonly known that these free radicals are highly reactive with membrane lipids, proteins, nucleic acids and other cellular substrates. It is also known that free radicals may be derived from environmental sources or may be generated in mammalian tissues. In order to protect these tissues as well as biological fluids from damages through free radicals, antioxidant enzymes are existing the activity of which is influenced by several co-factors.

Oxidative stress has been defined as "a disturbance in the pro-oxidant/antioxidant balance in favor of the former, leading to possible [tissue] damage" [Sies, H., Oxidative Stress. Oxidants and Antioxidants. 1991, New York: Elsevier. 507]. This balance can be related to one or more biochemical component of the biological fluid. Oxidative stress has been implicated as a key common pathway for cellular dysfunction and death and a potential therapeutic target in a broad spectrum of human medical conditions including cancer, diabetes, obstructive lung disease, inflammatory bowel disease, cardiac ischemia. glomerulonephritis, macular degeneration and various neurodegenerative disorders [Halliwell, B. and J.M.C. Gutteridge, Free Radicals in Biology and Medicine. 3 ed. 1999, Oxford: Oxford University Press Inc. 736].

Some of the end products of the cell/tissue damage, such as 3-nitrotyrosine for the nitration of proteins, 4-hydroxynonenal for the lipid peroxidation, or 8-hydroxyguanosine for nucleic acid damage, are already known, however, the detection processes are complicated and not sufficiently sensitive in order to detect gradual changes of the oxidative stress (for example, by therapeutic effects).

Therefore, it is an object underlying the present invention to provide for an improved method for determining an oxidative stress level in a biological sample which method is highly sensitive and allows for the detection of only slight changes in the oxidative stress level.

### Summary of the Invention

The present invention provides a method for determining an oxidative stress level in a biological sample as defined in claim 1 which comprises measuring the activity of one ore more enzymes which enzymes depend on one or more reducing co-factors, wherein the co-factor is tetrahydrobiopterin (BH4). Moreover, the activity of the enzymes is determined by measuring metabolite concentrations employing a quantitative analytical method such as chromatography, spectroscopy, and mass spectrometry. Particularly preferable is the use of the methods and devices as described in WO 2007/003344 and WO 2007/003343.

### Brief Description of the Figures

Figure 1 describes plasma phenylalanine concentrations of diabetic mice and healthy controls (Example 1);
Figure 2 describes plasma tyrosine concentrations of diabetic mice and healthy controls (Example 1);
Figure 3 describes plasma phenylalanine/tyrosine ratios of diabetic mice and healthy controls (Example 1);
Figure 4 describes increased methionine sulfoxide concentrations in high-grade (Gleason score > 8) compared to low-grade (Gleason score 6) prostate tumors indicating increased oxidative stress (Example 2);
Figure 5 describes increased tryptophan concentrations in high-grade (Gleason score > 8) compared to low-grade (Gleason score 6) prostate tumors indicating impaired activity of tryptophan hydroxylase due to oxidative stress (Example 2);
Figure 6 describes decreased serotonin concentrations in high-grade (Gleason score > 8) compared to low-grade (Gleason score 6) prostate tumors indicating impaired activity of tryptophan hydroxylase due to oxidative stress (Example 2);
Figure 7 describes increased tryptophan/serotonin ratio in high-grade (Gleason score > 8) compared to low-grade (Gleason score 6) prostate tumors indicating impaired activity of tryptophan hydroxylase due to oxidative stress (Example 2);
Figure 8 describes increasing methionine sulfoxide /methionine ratios indicating increasing oxidative stress during adaptation to high altitudes (Example 3); and
Figure 9 describes increasing phenylalanine / tyrosine ratios under increasing oxidative stress during adaptation to high altitudes indicating impaired activity of phenylalanine hydroxylase (Example 3).

### Description of the Preferred Embodiments

According to the method of the present invention an enzyme activity is measured in order to determine the oxidative stress level in a biological sample. Thus, the enzyme activity is a biomarker for the oxidative stress. It is an essential feature according to the invention that the activity of the enzymes under consideration depends on the presence of at least one anti-oxidative compound. Such a compound is also referred to as a reducing co-factor.

The invention is based on the finding that an elevated level of oxidative stress, i.e. the presence of more free radicals, causes increased oxidation of certain anti-oxidative compounds which are essential co-factors for several enzymes. The lack of sufficient quantities of these co-factors then causes a reduced enzymatic activity which is determined by measuring enzyme-dependent metabolite concentrations. In particular, the ratio of substrate/product of the enzyme is determined. An increase of said ratio (decreased enzyme activity) corresponds to an increased oxidative stress level and is, thus, a parameter potentially indicative for several diseases caused by an increased oxidative stress level.

The reducing co-factor is tetrahydrobiopterin (BH4). The biochemical function of BH4 as a member of the biopteridin redox system involved in the oxidation of aromatic rings is known in the art. It plays an essential role in the biosynthesis of biologically/biochemically important compounds, such as certain amino acids (e.g. tyrosine), catecholamins and serotonin.

The enzyme to be employed according to the invention is phenylalanine hydroxylase. The main biochemical functions of the enzyme mentioned above are also known in the art. It should be noted that the activity of this enzyme depends on the presence of the reducing co-factor BH4.

Specifically, the following metabolite concentrations (as the relevant substrate/product ratios) are determined in order to express the enzymes activities:
- the activity of phenylalanine hydroxylase is determined by measuring the phenylalanine/tyrosine ratio,

The biological sample usually is obtained from a mammal, preferably from a mouse, a rat, a guinea pig, a dog, a mini-pig, or a human. Thus, the method according to the invention is an *in vitro* method. For the measurement of the metabolite concentrations in the biological sample a quantitative analytical method such as chromatography, spectroscopy, and mass spectrometry is employed, while mass spectrometry is particularly preferred. The chromatography may comprise GC, LC, HPLC, and UPLC; spectroscopy may comprise UV/Vis, IR, and NMR; and mass spectrometry may comprise ESI-QqQ, ESI-QqTOF, MALDI-QqQ, MALDI-QqTOF, and MALDI-TOF-TOF. These analytical methods are generally known to the skilled person.

Potential therapeutic targets to be screened according to the method of the invention include a broad spectrum of human medical conditions such as various types of cancers, diabetes, obstructive lung disease, inflammatory bowel disease, cardiac ischemia, glomerulonephritis, macular degeneration and various neurodegenerative disorders. The method of the invention is also useful in detecting the gradual change of oxidative stress e.g. due to therapeutic effects.

Moreover the method of the invention may be used with a kit adapted for carrying out the method wherein the kit comprises a device which device contains one or more wells and one or more inserts impregnated with at least one internal standard. Such a device is in detail described in WO 2007/003344 and WO 2007/003343.

The invention will be described with respect to phenylalanine hydroxylase as follows. The underlying biochemical mechanism is described in more detail in the following Scheme. Phenylalanine hydroxylase converts phenylalanine into tyrosine in the presence of tetrahydrobiopterin (BH4). Under increased oxidative stress, i.e. the presence of more free radicals, tetrahydrobiopterin being a potent anti-oxidant itself is oxidized and thus depleted and, as a consequence thereof, is no longer present as the essential co-factor in a sufficient amount. The lack of sufficient quantities of this co-factor then causes a reduced enzymatic activity of the phenylalanine hydroxylase. Such a decreased enzymatic activity is indicated by an increased phenylalanine/tyrosine ratio and the observed accumulation of phenylalanine (in absolute terms and relative to tyrosine).

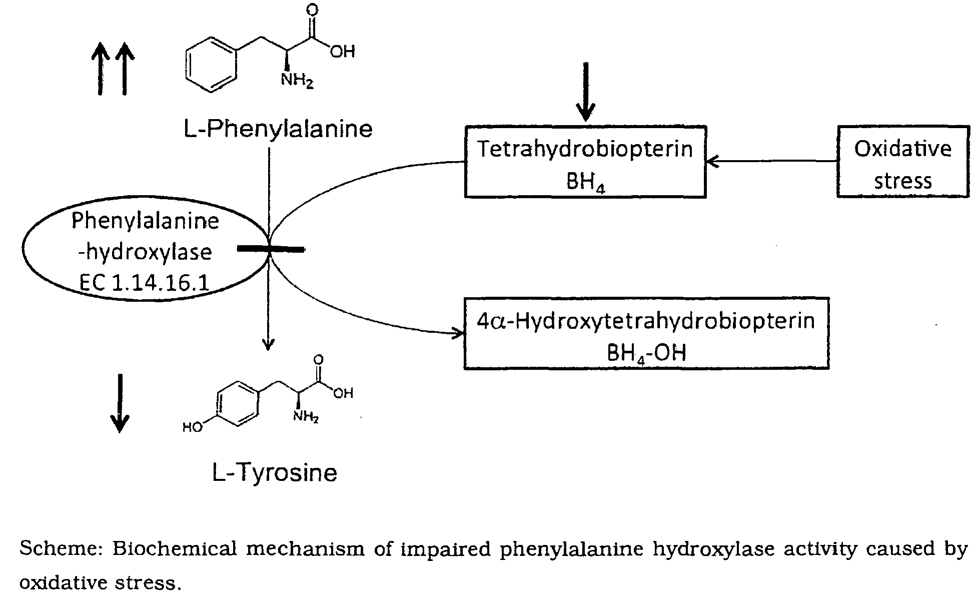

Even if the above mechanism is descrided herein for phenylalanine hydroxylase the present invention is not limited to measuring only the activity of phenylalanine hydroxylase. Quite in contrast, similar considerations also apply with respect to other enzymes which depend on BH4 or other reducing co-factors. This will become apparent from the following examples and reference examples.

### Example 1

Diabetes mellitus type II is a severe metabolic disease characterized by insulin-resistance of the liver and other tissues like skeletal muscle and adipose tissue. The primary symptom is an elevated glucose concentration in peripheral blood but accompanying findings include (non-enzymatic) glycation of proteins - frequently measured as glycated hemoglobin - and increasing dysregulation of many other metabolic pathways. Due to this wide range of pathophysiological alterations, the sequelae of diabetes mellitus type II range from cardiovascular problems with end-points like myocardial infarction or stroke to nephrological and neurological diseases and represent a huge socio-economic burden which is mainly caused by the continously increasing obesity of the population in the industrial countries.

One of the most important pathomechanisms in diabetes mellitus type II is a pronounced oxidative stress which immediately causes damage to important classes of biomolecules such as proteins, lipids and nucleic acids (see above). In this example plasma samples derived from a well-established mouse model for diabetes mellitus type II were analyzed, the db/db mouse with a homozygous genetic defect in the leptin receptor pathway leading to vastly increased food uptake, obesity, and the development of insulin resistance.

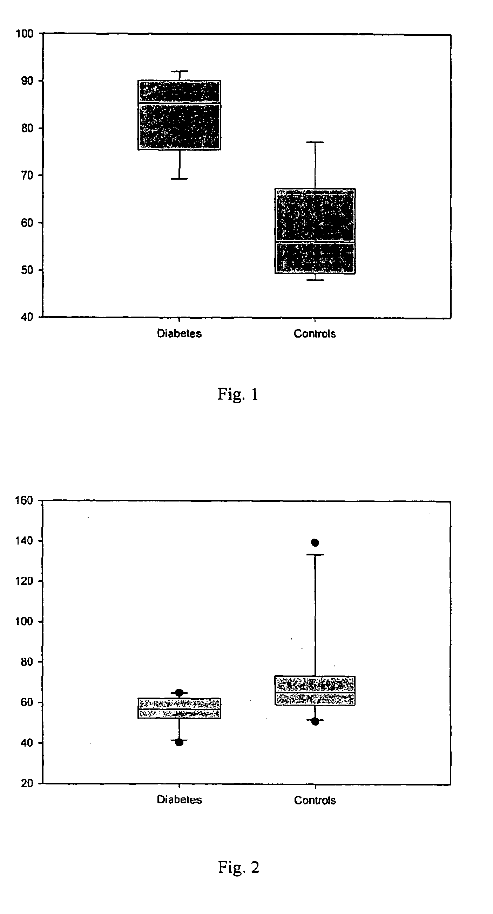

The plasma samples of the diabetic animals contained significantly elevated concentrations of the amino acid phenylalanine (82.2 ± 8.0 µM vs. 58.9 ± 9.7 µM; figure 1) while the concentrations of the amino acid tyrosine were slightly reduced (56.0 ± 7.1 µM vs. 51.5 ± 25.1 µM) (figure 2).

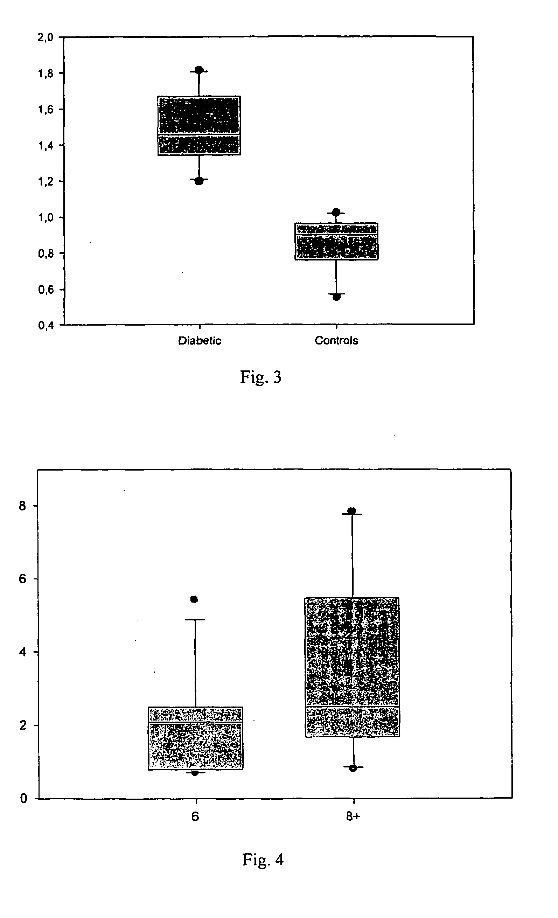

The critical ratio of phenylalanine to tyrosine concentrations indicative of the enzymatic activity of phenylalanine hydroxylase was, thus, significantly elevated in the diabetic cohort (1.48 ± 0.19 vs. 0.86 ± 0.14; figure 3).

### Example 2 (Reference)

The second example is chosen from an oncological indication. Prostate cancer ranges among the most frequent causes of death in males, in males over 75 years of age it is the most frequent cancer cause of death in the US. Although the actual causal mechanisms of prostate cancer are not well understood, at least some tumors are hormone-dependent (high testosterone levels increase tumor growth in these cases), and high-fat diet is described as an additional risk factor.

Oxidative stress has often been described to play an important role in prostate cancer, either as a causative agent (Kumar et al., Cancer Res 2008;68(6):1777-85) or as a mechanistic link between dietary factors and prostate cancer susceptibility (Fleshner & Klotz, Cancer Metastasis Rev. 1998-1999; 17(4):325-30).

In any case, oxidative stress is thought to occur in the hypoxic-ischemic core of solid tumors (Novotny et al., J Pediatr Surg. 2008 Feb;43(2):330-4). This seems paradoxal at first but other conditions which involve ischemia and hypoxia (e.g. coronary infarction, stroke etc.) also induce a marked oxidative stress in the affected tissue.

In this example, metabolic biomarkers for prostate cancer in clinical samples were studied; more specifically, tissue concentrations of various classes of metabolites were compared in patients with low-grade (Gleason score 6, n= 120) and high-grade (Gleason score >8, n = 85) prostate cancer which significantly influences the individual prognosis after radical prostatectomy.

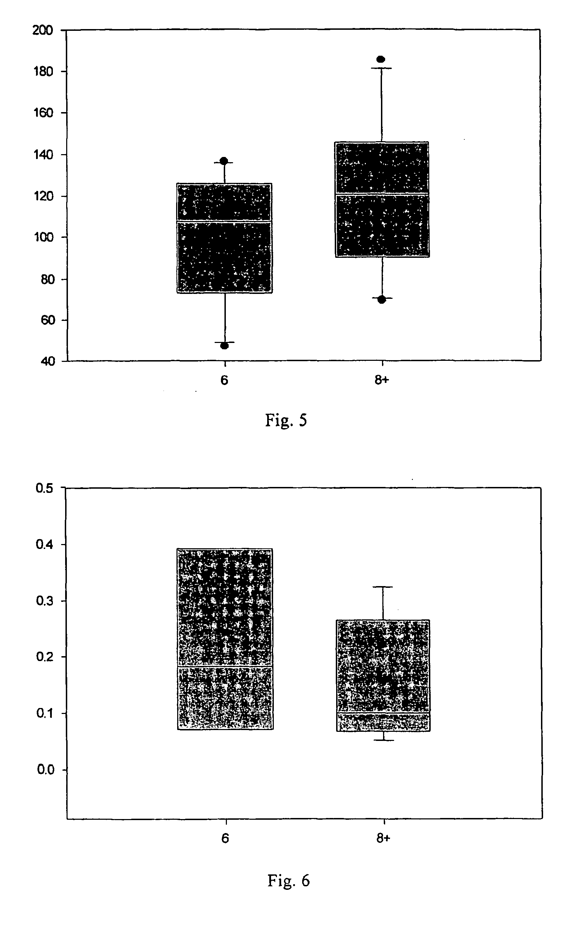

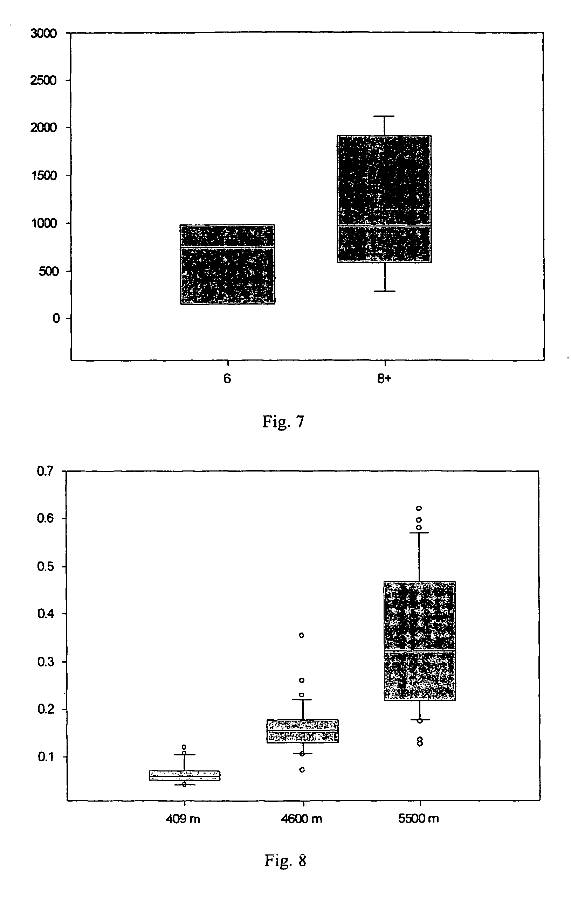

One of the main findings in this study was an elevated oxidative stress level in higher Gleason scores as demonstrated by increased concentrations of methionine sulfoxide (mean, 3.43 µM vs. 2.03 µM, figure 4). In the same comparison, an increase in tryptophan (mean, 119.0 µM vs. 101.3 µM, figure 5) and a decrease in serotonin (mean, 0.28 µM vs. 0.15 µM, figure 6) was identified. Moreover, the ratio of tryptophan to serotonin increased correspondingly (mean, 1169 vs. 999, figure 7) indicating an impaired enzymatic activity of tryptophan hydroxylase caused by depletion of tetrahydrobiopterin as an essential co-factor for this reaction.

### Example 3

As a third example, the adaptation of 33 healthy adult human volunteers to high altitudes during an expedition on the Muztagh Ata mountain in the Pamir region and the corresponding hypoxia-related metabolic alterations was analyzed. This hypoxia is again accompanied by increasing oxidative stress as demonstrated by drastically increasing ratios of methionine sulfoxide to methionine (figure 8).

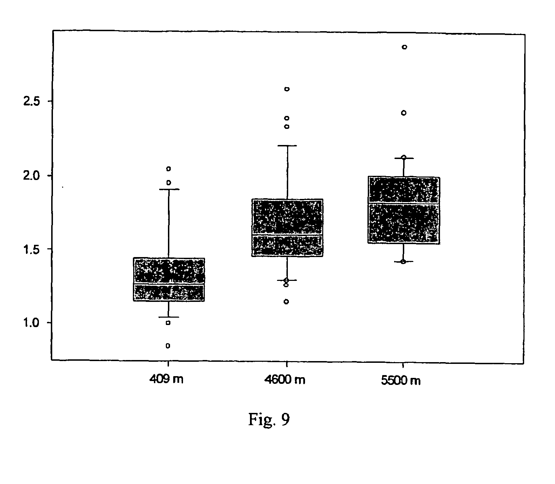

In analogy to the animal model used as the first example, an impaired activity of phenylalanine hydroxylase as measured by significantly increasing phenylalanine/ tyrosine ratios in this human study could also be demonstrated (figure 9).

### Industrial Applicability

The present invention provides for an Improved method for determining an oxidative stress level in a biological sample which method is highly sensitive and allows for the detection of only slight changes in the oxidative stress level. The method comprises the measurement of the activity of enzymes depending on at least one reducing co-factor (preferably BH4) as defined in claim 1. Namely, an elevated level of oxidative stress, i.e. the presence of more free radicals, causes increased oxidation of certain anti-oxidative compounds which are essential co-factors for several enzymes. The lack of sufficient quantities of these co-factors then causes a reduced enzymatic activity which is measured. Thus, the decreased enzyme activity is a biomarker for an increased oxidative stress level which allows the detection of several diseases and dysfunctions in mammals.

Potential therapeutic targets to be screened according to the method of the invention include a broad spectrum of human medical conditions such as various types of cancers, diabetes, obstructive lung disease, inflammatory bowel disease, cardiac ischemia, glomerulonephritis, macular degeneration and various neurodegenerative disorders. The method of the invention is also useful in detecting the gradual change of oxidative stress e.g. due to therapeutic effects. Thus, the method and the kit for carrying out the method are highly efficient in a lot of medical fields, both in diagnosis and therapy.

## Claims

1. A method for determining an oxidative stress level in a biological sample which comprises measuring the activity of one or more enzymes which depend on one or more reducing co-factors, wherein the co-factor is tetrahydrobiopterin and wherein the enzyme is phenylalanine hydroxylase.

2. The method according to claim 1, wherein the activity of phenylalanine hydroxylase is determined by measuring one or more metabolite concentrations.

3. The method according to claim 1, wherein the activity of phenylalanine hydroxylase is determined by measuring the phenylalanine/tyrosine ratio.

4. The method according to any one of claims 1 to 3, wherein the biological sample is obtained from a mammal, preferably from a mouse, a rat, a guinea pig, a dog, a mini-pig, or a human.

5. The method according to any one of claims 1 to 4, wherein the measurement is based on a quantitative analytical method, preferably chromatography, spectroscopy, and mass spectrometry.

6. The method according to claim 5, wherein chromatography comprises GC, LC, HPLC, and UPLC; spectroscopy comprises UV/Vis, IR, and NMR; and mass spectrometry comprises ESI-QqQ, ESI-QqTOF, MALDI-QqQ, MALDI-QqTOF, and MALDI-TOF-TOF.

## Patentansprüche

1. Verfahren zur Bestimmung des Niveaus an oxidativem Stress in einer biologischen Probe, welches die Messung der Aktivität eines oder mehrerer Enzyme, die von ein oder mehreren reduzierenden Co-Faktoren abhängen, umfasst, wobei der Co-Faktor Tetrahydrobiopterin ist und wobei das Enzym Phenylalaninhydroxylase darstellt.

2. Verfahren nach Anspruch 1, worin die Aktivität der Phenylalaninhydroxylase durch Messung einer oder mehrerer Metabolitenkonzentrationen bestimmt wird.

3. Verfahren nach Anspruch 1, worin die Aktivität der Phenylalaninhydroxylase durch Messung des Phenylalanin/Tyrosin-Verhältnisses bestimmt wird.

4. Verfahren nach einem der Ansprüche 1 bis 3, worin die biologische Probe erhalten wird von einem Säuger, vorzugsweise von einer Maus, einer Ratte, einem Meerschweinchen, einem Hund, einem Minischwein oder einem Menschen.

5. Verfahren nach einem der Ansprüche 1 bis 4, wobei die Messung auf einem quantitativen analytischen Verfahren beruht, vorzugsweise Chromatographie, Spektroskopie und Massenspektrometrie.

6. Verfahren nach Anspruch 5, wobei die Chromatographie GC, LC, HPLC und UPLC umfasst; die Spektroskopie UV/Vis, IR und NMR umfasst; und die Massenspektrometrie ESI-QqQ, ESI-QqTOF, MALDI-QqQ, MALDI-QqTOF und MALDI-TOF-TOF umfasst.

## Revendications

1. Procédé de détermination du niveau de stress oxydatif dans un échantillon biologique qui comprend la mesure de l'activité d'une ou de plusieurs enzymes qui dépendent d'un ou de plusieurs cofacteurs de réduction, dans lequel le cofacteur est la tétrahydrobioptérine et dans lequel l'enzyme est la phénylalanine hydroxylase.

2. Procédé selon la revendication 1, dans lequel l'activité de la phénylalanine hydroxylase est déterminée par mesure d'une ou de plusieurs concentrations de métabolites.

3. Procédé selon la revendication 1, dans lequel l'activité de la phénylalanine hydroxylase est déterminée par mesure du rapport phénylalanine/tyrosine.

4. Procédé selon l'une quelconque des revendications 1 à 3, dans lequel l'échantillon biologique est obtenu d'un mammifère, de préférence d'une souris, d'un rat, d'un cobaye, d'un chien, d'un mini-porc ou d'un humain.

5. Procédé selon l'une quelconque des revendications 1 à 4, dans lequel la mesure est basée sur un procédé analytique quantitatif, de préférence la chromatographie, la spectroscopie, et la spectrométrie de masse.

6. Procédé selon la revendication 5, dans lequel la chromatographie comprend la CG, la CL, la CLHP, et la CLUP ; la spectroscopie comprend l'UV/Vis, l'IR et la RMN ; et la spectrométrie de masse comprend l'ESI-QqQ, l'ESI-QqTOF, la MALDI-QqQ, la MALDI-QqTOF, et la MALDI-TOF-TOF.
